# EUROPÄISCHE PATENTSCHRIFT

(11) **EP 1 813 259 B1**
(45) Veröffentlichungstag und Bekanntmachung des Hinweises auf die Patenterteilung: **19.04.2017**
(21) Anmeldenummer: 06025549.4
(22) Anmeldetag: 11.12.2006
(51) Int. Cl.: A61K 8/44, A61K 8/64, A61K 8/49, A61K 8/34, A61Q 5/08, A61Q 5/10

(54) **Aufhell- und/oder Färbemittel mit reduziertem Irritationspotential**
Bleaching and/or colouring agent with reduced potential for irritation
Composition déclaircissement et/ou de coloration ayant un potentiel d'irritation réduit

(30) Priorität: 26.01.2006 DE 102006003924
(43) Veröffentlichungstag der Anmeldung: 01.08.2007
(73) Patentinhaber: Henkel AG & Co. KGaA, 40589 Düsseldorf (DE)
(72) Erfinder: Höffkes, Horst, 40595 Düsseldorf (DE); Brockmann, Claudia, 40627 Düsseldorf (DE)

(56) Entgegenhaltungen:
- EP-A1- 1 563 826
- EP-A2- 0 821 935
- WO-A1-96/23490
- WO-A2-98/11863
- WO-A2-2004/087086
- DE-A1- 3 541 485

## Beschreibung

Die vorliegende Erfindung betrifft Mittel zum Färben und/oder Aufhellen keratinischer Fasern, d.h. Mittel zur Anwendung auf Keratinfasern, insbesondere menschlichen Haaren.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und Blondierverfahren auf bestimmte Ausgangshaarfarben begrenzt. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

Gleichzeitig besteht aber auch vielfach der Wunsch, die Haarfarbe zu ändern und zusammen mit der Haarfärbung auch eine Aufhellung des zu färbenden Haares zu erreichen.

Konventionelle Haarfärbemittel bestehen in der Regel aus mindestens einer Entwickler- und mindestens einer Kupplersubstanz und enthalten ggf. noch direktziehende Farbstoffe als Nuanceure. Kuppler- und Entwicklerkomponenten werden auch als Oxidationsfarbstoffvorprodukte bezeichnet.

Vor ihrer Anwendung auf menschliches Haar werden Haarfärbe- und/oder -aufhellungsmittel in fester oder pastöser Form mit verdünnter wäßriger Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Ausfärbung bzw. Aufhellung liegt zwischen etwa 30 und 40 Minuten. Es ist naheliegend, daß bei den Benutzern dieser Haarfarben oder Blondiermittel ein Bedürfnis besteht, diese Einwirkungszeit zu verringern.

Weder die pastenförmigen noch die pulverförmigen Färbe- und/oder Blondiermittel, die heute auf dem Markt sind, können als optimal angesehen werden. Während die Färbe- und/oder Blondierwirkung auf dem Haar als auf die Verbraucherbedürfnisse zugeschnitten bezeichnet werden kann, bestehen doch noch eine Reihe von Nachteilen und Problemen sowohl bei Herstellung als auch bei der Handhabung dieser Mittel.

Nicht zuletzt durch die starke Beanspruchung der Haare, beispielsweise durch das Färben oder Dauerwellen als auch durch Umweltbelastungen, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein oder die Haare vor erhöhtem Spliß geschützt sein.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Färbung und/oder Aufhellung oder Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate wirken im allgemeinen bevorzugt an der Haaroberfläche. So sind Wirkstoffe bekannt, welche dem Haar Glanz, Halt, Fülle, bessere Naß- oder Trockenkämmbarkeiten verleihen oder dem Spliß vorbeugen. Genauso bedeutend wie das äußere Erscheinungsbild der Haare ist jedoch der innere strukturelle Zusammenhalt der Haarfasern, der insbesondere bei oxidativen und reduktiven Prozessen wie Färbung und Dauerwellen stark beeinflußt werden kann. Auch hierfür gibt es Wirkstoffe, die von ihrer Molekülgröße klein genug sind, um in den Cortex penetrieren zu können und zu einer innerstrukturellen Stabilisierung führen.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen.

Das Dokument EP 0821935 A2 beschreibt Mittel zum Färben und Tönen von Haaren auf der Basis von direktziehenden Farbstoffen, das einen sauern pH-Wert aufweist und mindestens ein nichtionisches Polymer sowie mindestens ein Proteinhydrolysat und/oder eine Aminosäure enthält.

In WO 98/11863 A2 werden Mittel zum Färben von Haaren beschrieben, die eine Kombination aus Bienenwachs mit mindestens einem Proteinhydrolysat und/oder mindestens einer Aminosäure enthalten.

Die Schrift DE 3541485 A1 offenbart Verfahren zu Behandlung von menschlichen Haaren mit einem alkalischen Haarbhehandlungsmittel, wobei die Haut vor oder nach der Behandlung der Haare mit dem alkalischen Haarbehandlungsmittel mit einem Vorbehandlungsmittel oder Nachbehandlungsmittel behandelt wird, das Valin und/oder einen Valinester enthält.

Das Dokument EP 1563826 A1 schließlich betrifft Verfahren zur Herstellung eines Keratinbehandlungsmittels, bei welchen mindesetns eine Aminosäure, ein Oligopeptid, ein Peptid, und ein Proteinhydrolysat zum Einsatz kommt.

Es wurde nun gefunden, daß sich bestimmte Wirkstoffkombinationen mit besonderem Vorteil in Haarfärbe- und -aufhellungsmittel einarbeiten lassen und zu einer Verringerung des Irritationspotentials dieser Mittel führen.

Gegenstand der Erfindung ist in einer ersten Ausführungsform ein Mittel zum Färben und/oder Aufhellen keratinischer Fasern, insbesondere menschlicher Haare, das, bezogen auf sein Gewicht
a) 0,05 bis 10 Gew.-% Valin;
b) 0,01 bis 10 Gew.-% Proteinhydrolysat(e)
enthält, dadurch gekennzeichnet, dass das/die Proteinhydrolysat(e) Molmassen im Bereich von 250 bis 50.000 Dalton aufweisen.

Die erfindungsgemäßen Mittel zeichnen sich durch eine gute Hautverträglichkeit aus, die sich sowohl auf der Kopfhaut als auch auf anderen Hautpartien, die mit dem Mittel in Berührung kommen, zeigt. Die erfindungsgemäßen Mittel verringern die irritative Wirkung der Mittel zum Färben und/oder Aufhellen keratinischer Fasern und schützen Haut und/oder Haare vor Überbeanspruchung durch diese Mittel. Darüber hinaus wird die Hautverträglichkeit von Mitteln zum Färben und/oder Aufhellen keratinischer Fasern gesteigert und die haarstrukturschädigende Wirkung von diesen Mitteln verringert. Nicht zuletzt zeigen die erfindungsgemäßen Mittel auch einen haarstrukturverbessernden Effekt auf den mit ihnen behandelten Haaren.

Die erfindungsgemäßen Mittel enthalten als ersten wesentlichen Inhaltsstoff Valin. Das erfindungsgemäß eingesetzte Valin kann dabei als Racemat oder auch in Form einer seiner optischen Isomeren in die Mittel eingearbeitet werden. Neben dem Racemat ist das natürlich vorkommende Valin [(S)-2-Amino-3-methylbuttersäure bzw. (*S*)-alpha-Aminoisovaleriansäure] bevorzugt. Bevorzugte erfindungsgemäße Mittel enthalten ausschließlich S-Valin.

Die Synthese von Valin kann durch Aminierung von 2-Brom-3-methylbuttersäure erfolgen; zur gegebenenfalls gewünschten enzymatischen Racemattrennung wird vorzugsweise das *N*-Acetyl-Derivat eingesetzt. Valin kann auch werden durch Extraktion von Eiweißhydrolysaten und durch Fermentation mit geeigneten Bakterienmutanten gewonnen werden. Bei der Fermentation werden überwiegend Glucose-Lösungen eingesetzt und die größten Ausbeuten mit bestimmten Mutanten von *Corynebacterium glutamicum* bzw. *Brevibacterium lactofermentum* erzielt.

Erfindungsgemäß bevorzugte Mittel zum Färben und/oder Aufhellen keratinischer Fasern sind dadurch gekennzeichnet, daß sie - bezogen auf ihr Gewicht - 0,1 bis 7,5 Gew.-%, vorzugsweise 0,15 bis 5 Gew.-%, besonders bevorzugt 0,25 bis 3,5 Gew.-% und insbesondere 0,5 bis 2 Gew.-% Valin enthalten.

Als zweiten wesentlichen Bestandteil enthalten die erfindungsgemäßen Mittel 0,01 bis 10 Gew.-% Proteinhydrolysat(e).

Als Proteine, aus denen die Proteinhydrolysate gewonnen werden können, kommen im Rahmen der vorliegenden Erfindung grundsätzlich alle Proteine unabhängig von ihrem Ursprung oder ihrer Herstellung in Betracht. Beispiele für Proteine tierischen Ursprungs sind Keratin, Elastin, Kollagen, Fibroin, Albumin, Casein, Molkeprotein, Plazentaprotein. Erfindungsgemäß bevorzugt aus diesen sind Kollagen, Keratin, Casein, Molkeprotein, Proteine pflanzlichen Ursprungs wie beispielsweise Weizen- und Weizenkeimprotein, Reisprotein, Sojaprotein, Haferprotein, Erbsenprotein, Kartoffelprotein, Mandelprotein und Hefeprotein können erfindungsgemäß ebenfalls bevorzugt sein.

Unter Proteinhydrolysaten sind im Rahmen der vorliegenden Erfindung Abbauprodukte von Proteinen wie beispielsweise Kollagen, Elastin, Casein, Keratin, Mandel-, Kartoffel-, Weizen-, Reis- und Sojaprotein zu verstehen, die durch saure, alkalische und / oder enzymatische Hydrolyse der Proteine selbst oder ihrer Abbauprodukte wie beispielsweise Gelatine erhalten werden. Für den enzymatischen Abbau sind alle hydrolytisch wirkenden Enzyme geeignet, wie z. B. alkalische Proteasen.

Weiterhin sind im Rahmen der vorliegenden Erfindung als Proteinhydrolysate bevorzugt die in der Kosmetik gebräuchlichen Proteinhydrolysate mit einem durchschnittlichen Molekulargewicht im Bereich von 600 bis 4000, besonders bevorzugt von 2000 bis 3500. Übersichten zu Herstellung und Verwendung von Proteinhydrolysaten sind beispielsweise von G. Schuster und A. Domsch in Seifen Öle Fette Wachse **108,** (1982) 177 bzw. Cosm.Toil. **99,** (1984) 63, von H. W. Steisslinger in Parf.Kosm. **72,** (1991) 556 und F. Aurich et al. in Tens.Surf.Det. **29,** (1992) 389 erschienen. Vorzugsweise werden erfindungsgemäß Proteinhydrolysate aus Kollagen, Keratin, Casein sowie pflanzlichen Proteinen eingesetzt, beispielsweise solche auf Basis von Weizengluten oder Reisprotein.

Weitere Beispiele für Proteinhydrolysate und Proteinhydrolysat-Derivate, die unter den Rahmen der vorliegenden Erfindung fallen, sind beschrieben in CTFA 1997 International Buyers' Guide, John A. Wenninger et al. (Ed.), The Cosmetic, Toiletry, and Fragrance Association, Washington DC 1997, 686-688.

Erfindungsgemäß bevorzugte Mittel zum Färben und/oder Aufhellen keratinischer Fasern sind dadurch gekennzeichnet, daß sie - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 1,5 Gew.-% Proteinhydrolysat(e) enthalten.

Weiter erfindungsgemäß ist es, in den erfindungsgemäßen Mitteln Proteinhydrolysate eines bestimmten Molmassenbereiches einzusetzen. Hier sind Mittel zum Färben und/oder Aufhellen keratinischer Fasern erfindungsgemäß bei denen das/die Proteinhydrolysat(e) Molmassen im Bereich von 250 bis 50.000 und insbesondere von 500 bis 20.000 Dalton. aufweisen.

Unter den Proteinhydrolysaten - und besonders unter denen des vorstehend genannten Momlassenbereiches - sind bestimmte Proteinhydrolysate bevorzugt. In bevorzugten erfindungsgemäßen Mitteln zum Färben und/oder Aufhellen keratinischer Fasern ist/sind das/die in ihm enthaltene(n) Proteinhydrolysat(e) ausgewählt ist/sind aus Hydrolysaten von tierischen und/oder pflanzlichen Proteinen, vorzugsweise aus
- Milchproteinhydrolysaten
- Keratinhydrolysaten
- Weizenproteinhydrolysaten
- Maisproteinhydrolysaten
- Reisproteinhydrolysaten
- Kartoffelproteinhydrolysaten
- Sojaproteinhydrolysaten
- Mandelproteinhydrolysaten
- Algenproteinhydrolysaten.

Die erfindungsgemäßen Mittel können zusätzlich zu Proteinhydrolysaten oder an ihrer Stelle auch Derivate von Proteinhydrolysaten enthalten. Unter Proteinhydrolysat-Derivaten sind im Rahmen der vorliegenden Erfindung chemisch und / oder chemoenzymatisch modifizierte Proteinhydrolysate zu verstehen wie beispielsweise die unter den INCI-Bezeichnungen Sodium Cocoyl Hydrolyzed Wheat Protein, Laurdimonium Hydroxypropyl Hydrolyzed Wheat Protein, Potassium Cocoyl Hydrolyzed Collagen, Potassium Undecylenoyl Hydrolyzed Collagen und Laurdimonium Hydroxypropyl Hydrolyzed Collagen bekannten Verbindungen. Vorzugsweise werden erfindungsgemäß Derivate aus Proteinhydrolysaten des Kollagens, Keratins und Caseins sowie pflanzlichen Proteinhydrolysaten eingesetzt wie z. B. Sodium Cocoyl Hydrolyzed Wheat Protein oder Laurdimonium Hydroxypropyl Hydrolyzed Wheat Protein.Besonders bevorzugt sind dabei kationisierte Proteinhydrolysate.

Bevorzugte erfindungsgemäße Mittel zum Färben und/oder Aufhellen keratinischer Fasern sind dadurch gekennzeichnet, daß sie kationische Proteinhydrolysate enthalten, wobei bevorzugte Mittel ein oder mehrere kationische Proteinhydrolysate aus der Gruppe Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein enthalten.

Eine weitere bevorzugte Gruppe von Inhaltsstoffen der erfindungsgemäßen Mittel sind Vitamine, Provitamine oder Vitaminvorstufen. Diese werden nachfolgend beschrieben:
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.
Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Mittel zum Färben und/oder Aufhellen keratinischer Fasern bevorzugt , die zusätzlich Vitamine und/oder Pro-Vitamine und/oder Vitaminvorstufen enthalten, die vorzugsweise den Gruppen A, B, C, E, F und H zugeordnet werden, wobei bevorzugte Mittel die genannten Verbindungen in Mengen von 0,1 bis 5 Gew.-%, vorzugsweise von 0,25 bis 4 Gew.-% und insbesondere von 0,5 bis 2,5 Gew.-%, jeweils bezogen auf das gesamte Mittel, enthalten.

Erfindungsgemäß bevorzugte Mittel enthalten zusätzlich mindestens ein 2-Furanonderivat der Formel (I) und/oder der Formel (II). 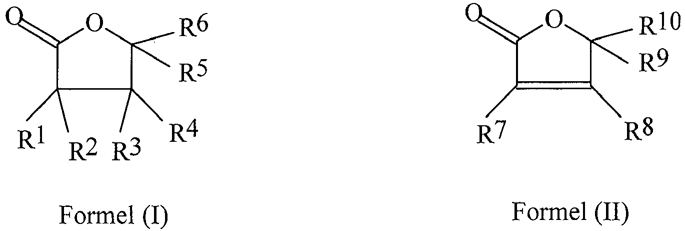 in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen-C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

2-Furanone sind bekannte Verbindungen und werden beispielsweise in "Römpps Lexikon der Chemie, Interactive CD-Rom Version 2.0, zum Stichwort "Dihydro-3-hydroxy-4,4-dimethyl-2(3H)-furanon" sowie in "Ullmann's Encyclopedia, sixth edition 1999, electronic release" in den Abschnitten 2.4, 2.7, 3.2, 3.4, 4.3, 6., 11. und 15. und den dort zitierten Schriften bezüglich der Herstellung und Verwendung beschrieben. Auf diese Kapitel und die dort zitierte Literatur wird ausdrücklich Bezug genommen. Die Verbindungen der Formeln (I) und (II) werden als Zwischenstufen in der Naturstoffsynthese sowie der Herstellung von Arzneimitteln und Vitaminen eingesetzt. Die Herstellung der Wirkstoffe gemäß der Formeln (I) und (II) kann beispielsweise durch Umsetzung von primären Alkoholen mit Acrylsäuren erfolgen. Weiterhin gelangt man zu Verbindungen der Formel (I) durch Reaktionen ausgehend von Hydroxypivaldehyd. Ebenfalls führen Carbonylierungen von Alkynen zu substituierten 2-Furanonen der Formel (I) oder (II).

Schließlich können die Verbindungen der Formel (I) oder der Formel (II) durch intramolekulare Veresterung der entsprechenden Hydroxycarbonsäuren erhalten werden. Beispielsweise werden die folgenden Verbindungen auf einem der zuvor aufgezeigten Synthesewege erhalten: 2,5-Dihydro-5-methoxy-2-furanon,
Tetrahydro-5-oxo-2-furancarbonsäure, Dihydro-3-hydroxy-4,4-dimethyl-2(3H)-furanon, oder 3,4-Dimethyl-5-pentylidenedihydro-2(5H)-furanon oder 4-Hydroxy-2,5-dimethyl-3(2H)-furanon. Die erfindungsgemäßen 2-Furanone umfassen selbstverständlich alle möglichen Stereoisomere wie auch deren Gemische. Durch die erfindungsgemäßen 2-Furanone wird der Geruch der kosmetischen Mittel nicht nachhaltig beeinflußt, so daß eine Parfümierung der Mittel separat erfolgen muß.

Bevorzugte Verbindungen der Formel (I) und/oder der Formel (II) können Verbindungen sein, bei welchen die Substituenten R¹, R² und R⁷ unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- einen -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
   - eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
   - eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
   - eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen - C₂ - C₃₀ - gesättigten oder ein- oder mehrfach ungesättigten, verzweigten oder linearen Mono-, Di-, Tri- oder Polyhydroxyalkylrest, oder einen -C₂ - C₃₀ - gesättigten oder ein- oder mehrfach ungesättigten, verzweigten oder linearen Mono-, Di-, Tri- oder Polyaminokohlenwasserstoffrest.

In einer weiteren Ausführungsform der erfindungsgemäßen Lehre hat es sich gezeigt, daß bei den Verbindungen der Formel (I) oder der Formel (II) die Reste R³, R⁴ und R⁸ bevorzugt unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Kohlenwasserstoffrest,
- einen -C₂ - C₄ - gesättigten oder ein- oder zweifach ungesättigten, verzweigten oder linearen Mono-, Di- oder Trihydroxykohlenwasserstoffrest oder
- einen -C₂ - C₄ - gesättigten oder ein- oder zweifach ungesättigten, verzweigten oder linearen Mono-, Di- oder Triaminokohlenwasserstoffrest.
Weiterhin kann es bevorzugt sein, wenn in dem erfindungsgemäßen Wirkstoff gemäß der Formel(I) und/oder der Formel(II) für die Reste R⁵, R⁶, R⁹ und R¹⁰ unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Kohlenwasserstoffrest,
- einen -C₂ - C₄ - gesättigten oder ein- oder zweifach ungesättigten, verzweigten oder linearen Mono-, Di- oder Trihydroxykohlenwasserstoffrest oder
- einen -C₂ - C₄ - gesättigten oder ein- oder zweifach ungesättigten, verzweigten oder linearen Mono-, Di- oder Triaminokohlenwasserstoffrest.

In einer besonders bevorzugten Ausführungsform der erfindungsgemäßen Lehre wird eine Verbindung der Formel (I) eingesetzt. Dabei kann es bevorzugt sein, daß in einer Verbindung der Formel (I) die Reste R¹ und R² unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen - C₂ - C₃₀ - gesättigten oder ein- oder mehrfach ungesättigten, verzweigten oder linearen Mono-, Di-, Tri- oder Polyhydroxykohlenwasserstoffrest.

Weiterhin kann es in dieser besonders bevorzugten Ausführungsform der erfindungsgemäßen Lehre vorteilhaft sein, wenn in den Verbindungen der Formel (I) die Reste R³ und R⁴ unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- und/oder Polyhydroxykohlenwasserstoffrest.

In dieser bevorzugten Ausführungsform kann es weiterhin vorteilhaft sein, daß die Verbindungen gemäß Formel (I) für die Reste R5 und R6 unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest.

In einer besonders bevorzugten Ausführungsform der erfindungsgemäßen Lehre wird als Verbindung entsprechend der Formel (I)
- (R)-(-)-4-Hydroxymethyl-γ-butyrolacton und/oder
- D,L-4-Hydroxymethyl-γ-butyrolacton und/oder
- (S)-(+)-4-Hydroxymethyl-γ-butyrolacton und/oder
- R-(-)-2-Hydroxy-3,3-dimethyl-γ-butyrolacton und/oder
- D,L-2-Hydroxy-3,3-dimethyl-γ-butyrolacton und/oder
- S(+)-2-Hydroxy-3,3-dimethyl-γ-butyrolacton und/oder
- 4-Hydroxy-2,5-dimethyl-3(2H)-furanon und/oder
- Tetrahydro-5-oxo-2-furancarbonsäure und/oder
- Tetrahydro-5-oxo-2-furancarbonsäure, Na-Salz und/oder
- Tetrahydro-5-oxo-2-furancarbonsäure, K-Salz und/oder
- 2,5-Dihydro-5-methoxy-2-furanon und/oder
- Dihydro-3-hydroxy-4,4-dimethyl-2(3*H*)-furanon
eingesetzt. In einer ganz besonders bevorzugten Ausführungsform der erfindungsgemäßen Lehre wird als Verbindung entsprechend der Formel (I) Dihydro-3-hydroxy-4,4-dimethyl-2(3H)-furanon eingesetzt.

Zusammenfassend sind bevorzugt erfindungsgemäße Mittel zum Färben und/oder Aufhellen keratinischer Fasern dadurch gekennzeichnet, daß sie zusätzlich 0,05 bis 15 Gew.-% mindestens eines 2-Furanonderivats der Formel (I) und/oder der Formel (II) 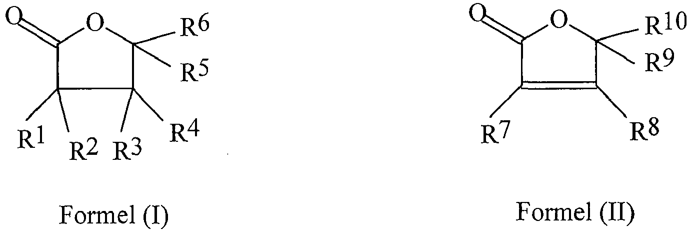 enthalten, in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen - C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

Bevorzugt ist auch der zusätzlich Einsatz von Bisabolol und/oder Bisabololoxiden in den erfindungsgemäßen Mitteln. Hier sind erfindungsgemäße Mittel zum Färben und/oder Aufhellen keratinischer Fasern bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol 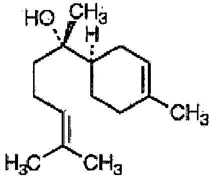 enthalten.

Die erfindungsgemäßen Mittel können als Mittel zum Färben und/oder als Mittel zum Aufhellen keratinischer Fasern konfektioniert werden. Unter keratinischen bzw. keratinhaltigen Fasern werden im Rahmen dieser Anmeldung Pelze, Wolle, Federn und insbesondere menschliche Haare verstanden.

Es ist also erfindungsgemäß möglich, ein Färbemittel (sogenanntes "Oxidationsfärbemittel") bereitzustellen, oder ein reines Blondiermittel (auch Aufhellmittel genannt). Selbstverständlich sind auch Produkte, die gleichzeitig aufhellen und färben, erfindungsgemäß herstellbar.

Die erfindungsgemäßen Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können die erfindungsgemäßen Oxidationsfärbemittel auch noch direktziehende Farbstoffe als Nuanceure enthalten.
Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.
Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Hinsichtlich der in den erfindungsgemäßen Färbemitteln einsetzbaren weiteren Farbstoffvorprodukte unterliegt die vorliegende Erfindung keinerlei Einschränkungen. Die erfindungsgemäßen Färbemittel können als weitere Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.
In einer ersten bevorzugten Ausführungsform enthält das Färbemittel weiterhin mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Erfindungsgemäß ganz besonders bevorzugte p-Phenylendiaminderivate sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Ganz besonders bevorzugte zweikernige Entwicklerkomponenten sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-PyrimidinDerivaten und ihren physiologisch verträglichen Salzen.
Bevorzugte Pyridin-Derivate sind insbesondere die Verbindungen 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Unter den Pyrazol-[1,5-a]-pyrimidinen kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)amino]-ethanol;
- 2-[(7-Aminopyrazol-[1,5-a]-pyrimidin-3-yl)-(2-hydroxy-ethyl)amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Erfindungsgemäß besonders bevorzugte Oxidationsfärbemittel sind dadurch gekennzeichnet, daß die Entwicklerkomponente ausgewählt ist aus 3-Methyl-1,4-diaminobenzol, 1-(2'-Hydroxyethyl)-2,5-diaminobenzol, 2-(2,5-Diaminophenoxy)-ethanol, N,N-Bis(2'-Hydroxyethyl)-1,4-diaminobenzol, 3-Methyl-4-aminophenol und 2-Methylamino-4-aminophenol, p-Phenylendiamin, 2-(ß-Hydroxyethyl)-p- phenylendiamin, N,N-Bis-(β-hydroxyethyl)-p-phenylendiamin, N,N'-bis-(ß-Hydroxyethyl)-N,N'-bis- (4'-aminophenyl)-1,3-diamino-propan-2-ol, Bis-(2-hydroxy-5-aminophenyl)- methan, N,N'-Bis-(4'-aminophenyl)-1,4-diazacycloheptan, 1,10-Bis-(2',5'-diaminophenyl)-1,4,7,10-tetraoxadecan, p-Aminophenol, 4- Amino-3-fluorphenol, 4-Amino-2-aminomethylphenol, 4- Amino-2- ((diethylamino)methyl)phenol, o-Aminophenol, 2-Amino-4-methylphenol, 2-Amino-5- methylphenol, 2-Amino-4-chlorphenol, 2,4,5,6- Tetraaminopyrimidin, 4-Hydroxy-2,5,6- triaminopyrimidin, 2-Hydroxy-4,5,6- triaminopyrimidin, 2-Dimethylamino-4,5,6-triaminopyrimidin, 2,4- Dihydroxy-5,6-diaminopyrimidin, 2,5,6-Triaminopyrimidin, 1-(2'- Hydroxy- 5'-aminobenzyl)-imidazolidin-2-on, 4,5-Diamino-1-(2'-hydroxyethyl) pyrazol und *N*-(4-Amino-3-methylphenyl)-*N*-[3-(1*H*-imidazol-1-yl)propyl]amin trihydrochlorid.
Die erfindungsgemäßen Färbemittel enthalten mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Erfindungsgemäß besonders bevorzugte Oxidationsfärbemittel sind dadurch gekennzeichnet, daß die Kupplerkomponente ausgewählt ist aus m-Phenylendiaminderivaten, Naphtholen, Resorcin und Resorcinderivaten, Pyrazolonen, m-Aminophenolen und substituierten Pyridinderivaten, wobei bevorzugte Mittel Resorcin, 3-Amino-2-methylamino-6-methoxypyridin, 3-Amino-6-methylphenol, 3-Amino-2-hydroxypyridin, 1,3-Bis-(2,4-diaminophenoxy)propan, 2,7-Dihydroxynaphthalin, 2-Methylresorcin, 5-Methyl-resorcin, 2,5-Dimethylresorcin und 4-Chlorresorcin 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 3-Amino-4-(2-methoxyethoxy)-5-methylphenylamin und/oder 2-[(3-Morpholin-4-ylphenyl)amino]ethanol dihydrochlorid enthalten.

Vorzugsweise werden Kuppler- und Entwicklerkomponenten in einem bestimmten Verhältnis zueinander eingesetzt. Hier sind erfindungsgemäße Oxidationsfärbemittel bevorzugt, die die Kupplerkomponente(n) in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf das gesamte Oxidationsfärbemittel, enthalten.

Zusätzlich können die Färbemittel zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Erfindungsgemäß bevorzugte Oxidationsfärbemittel sind dadurch gekennzeichnet, daß sie mindestens einen direktziehenden Farbstoff enthaltender ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

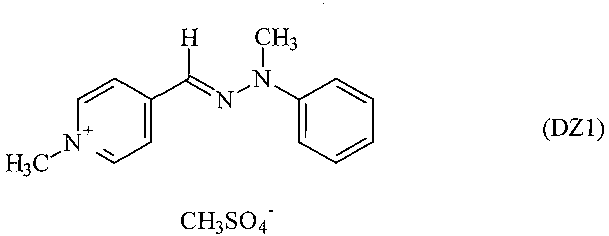

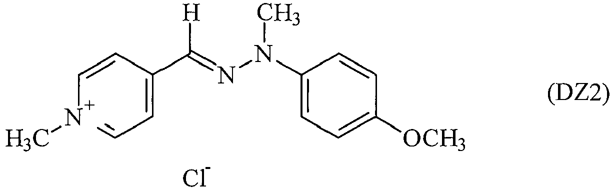

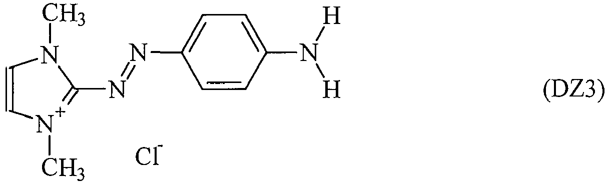

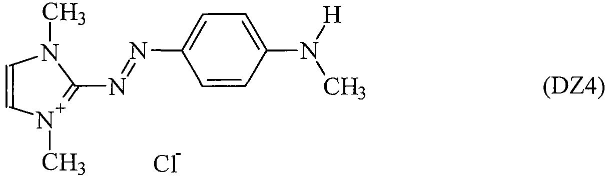

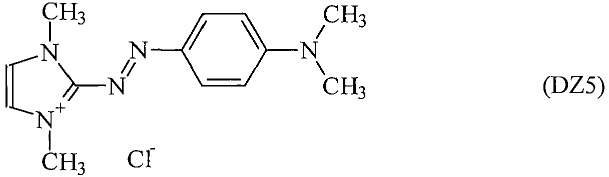

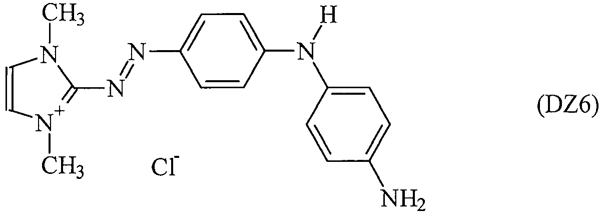

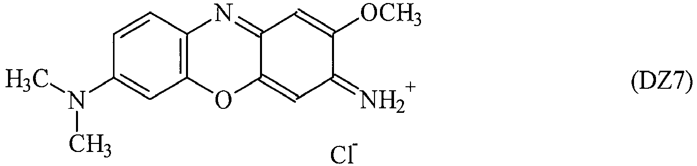

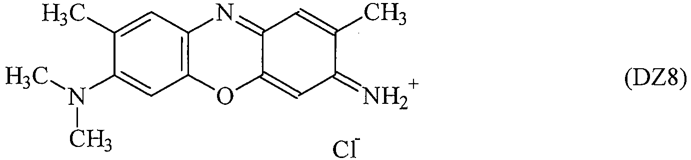

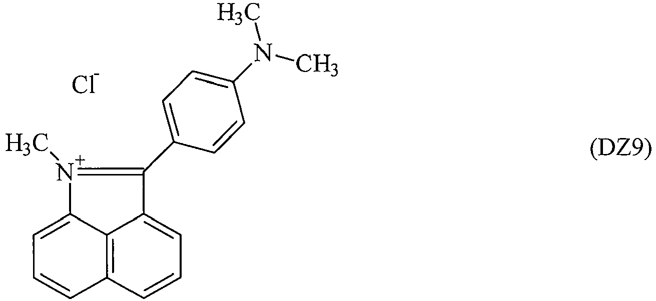

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Mittel werden vorzugsweise unmittelbar vor ihrer Anwendung durch Vermischen zweier getrennt verpackter Zubereitungen hergestellt, wobei eine Zubereitung Farbstoffvorprodukt(e) sowie gegebenenfalls direktziehende Farbstoffe enthält und/oder eine Blondiercreme ist und die zweite Zubereitung eine - vorzugsweise wäßrige - Oxidationsmittelzubereitung ist.

Ein weiterer Gegenstand der vorliegenden Erfindung ist daher ein Mittel zum Färben und/oder Aufhellen keratinischer Fasern, insbesondere menschlicher Haare, welches unmittelbar vor dem Aufbringen auf das Haar aus einer fließfähigen Zubereitung A und einer Oxidationsmittelzubereitung B, enthaltend mindestens ein Oxidationsmittel, ausgewählt aus Wasserstoffperoxid und dessen Anlagerungsverbindungen an feste Träger, die miteinander im Gewichtsverhältnis 1:4 bis 4:1 zu einem Färbe- und/oder Aufhellansatz vermischt werden, erhalten wird, wobei die fließfähige Zubereitung A - bezogen auf ihr Gewicht -
a. 0,1 bis 20 Gew.-% Valin;
b. 0,02 bis 20 Gew.-% Proteinhydrolysat(e) enthält.

Die Oxidationsmittelzubereitung B enthält mindestens ein Oxidationsmittel, ausgewählt aus Wasserstoffperoxid und dessen Anlagerungsverbindungen an feste Träger. Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon.n H₂O₂ (n ist eine positive ganze Zahl größer 0), Harnstoffperoxid und Melaminperoxid, eingesetzt.

Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet.

Die Oxidationsmittelzubereitung B ist vorzugsweise eine wäßrige, fließfähige Oxidationsmittelzubereitung.

Vorzugsweise wird der fließfähigen Oxidationsmittelzubereitung B ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind.

Als anionische Tenside und Emulgatoren eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (VIII), 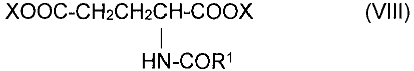 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (IX), 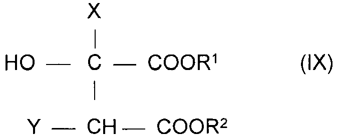 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobernsteinsäure oder der Sulfosuccinate der allgemeinen Formel (X), 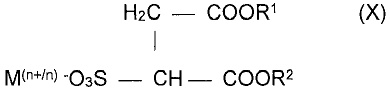 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside und Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.
Als zwitterionische Tenside und Emulgatoren werden solche oberflächenaktiven Verbindungen bezeichnet, die im Molekül mindestens eine quartäre Ammoniumgruppe und mindestens eine-COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside und Emulgatoren enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie Hydagen^{®} HSP (Cognis) oder Sovermol^{®} - Typen (Cognis), insbesondere von gesättigten C₈₋₃₀-Fettsäuren,
- alkoxylierte Triglyceride,
- alkoxylierte Fettsäurealkylester,
- Aminoxide,
- Fettsäurealkanolamide, Fettsäure-N-alkylglucamide und Fettamine sowie deren Ethylenoxid- oder Polyglycerin-Anlagerungsprodukte,
- Sorbitanfettsäureester und Anlagerungsprodukte von Ethylenoxid an Sorbitanfettsäureester wie beispielsweise die Polysorbate,
- Zuckerfettsäureester und Methylglucosid-Fettsäureester sowie deren Ethylenoxid- oder Polyglycerin-Anlagerungsprodukte,
- Alkylpolyglycoside entsprechend der allgemeinen Formel RO-(Z)ₓ wobei R für Alkyl, Z für Zucker sowie x für die Anzahl der Zuckereinheiten steht. Besonders bevorzugt sind solche Alkylpolyglycoside, bei denen R
   - im wesentlichen aus C₈- und C₁₀-Alkylgruppen,
   - im wesentlichen aus C₁₂- und C₁₄-Alkylgruppen,
   - im wesentlichen aus C₈- bis C₁₆-Alkylgruppen oder
   - im wesentlichen aus C₁₂- bis C₁₆-Alkylgruppen oder
   - im wesentlichen aus C₁₆ bis C₁₈-Alkylgruppen
besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.
- Gemische aus Alkyl-(oligo)-glucosiden und Fettalkoholen, z. B. Montanov^{®}68,
- Sterine, z. B. Ergosterin, Stigmasterin, Sitosterin und Mykosterine,
- Phospholipide, z. B. Lecithine bzw. Phosphatidylcholine,
- Polyglycerine und Polyglycerinderivate wie beispielsweise Polyglycerinpoly-12-hydroxystearat (Dehymuls^{®} PGPH) oder Triglycerindüsostearat (Lameform^{®} TGI),
- alkoxylierte Polydialkylsiloxane (INCI-Bezeichnung: Dimethicone Copolyol).

Als bevorzugte nichtionische oberflächenaktive Substanzen haben sich die Alkylpolyglycoside, gegebenenfalls im Gemisch mit Fettalkoholen, alkoxylierte Polydialkylsiloxane, Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Ganz besonders bevorzugt sind erfindungsgemäße Mittel zum Färben und/oder Aufhellen keratinischer Fasern, bei denen die fließfähige Oxidationsmittelzubereitung B zusätzlich Fettalkohol(e) und/oder Fettalkoholalkoxylat(e), vorzugsweise C₁₂₋₂₂-Fettalkohol(e) und/oder C₁₂₋₂₂-Fettalkoholethoxylat(e) mit 10 bis 30 EO-Einheiten, besonders bevorzugt C₁₆₋₁₈-Fettalkohol(e) und/oder C₁₆₋₁₈-Fettalkoholethoxylat(e) mit 12 bis 20 EO-Einheiten, vorzugsweise in Mengen von 5 bis 20 Gew.-%, bevorzugt von 7,5 bis 17,5 Gew.-% und insbesondere von 10 bis 15 Gew.-%, jeweils bezogen auf das Gewicht der fließfähigen Oxidationsmittelzubereitung B, enthält.

Die erfindungsgemäßen Haarfärbe- und/oder -aufhellungsmittel können als Kombinationspräparate (Färbung und gleichzeitige Aufhellung), als reine Färbemittel oder als reine Aufhellungsmittel (Blondiermittel) formuliert werden. Je nach gewünschtem Einsatzzweck enthalten die erfindungsgemäßen Mittel dann weitere Inhaltsstoffe, die nachfolgend detailliert beschrieben werden.

Eine bevorzugte Ausführungsform erfindungsgemäßer Mittel stellen die Blondiermittel dar. Diese werden vorzugsweise als Pasten konfektioniert, um beispielsweise dem Problem der Staubentwicklung bei der Anwendung Rechnung zu tragen. Ein bevorzugtes erfindungsgemäßes Mittel ist daher dadurch gekennzeichnet, daß es als Blondierpaste formuliert ist, die bezogen auf ihr Gewicht 1 bis 50 Gew.-%, vorzugsweise 10 bis 45 Gew.-% und insbesondere 20 bis 40 Gew.-% Oxidationsmittel, vorzugsweise in Form ein oder mehrerer Peroxidisulfat(e) enthält.

Die Bezeichnung "Paste" ist ein nicht scharf definierter Begriff für Festkörperdispersionen in Flüssigkeiten von teigiger Konsistenz, der im Rahmen der vorliegenden Erfindung weit auszulegen ist.

Die erfindungsgemäßen Blondiermittel enthalten ein Oxidationsmittel, vorzugsweise eine Peroxoverbindung. In der in den erfindungsgemäßen Mitteln enthaltenen fließfähigen Oxidationsmittelzubereitung B ist Wasserstoffperoxid enthalten, wobei weitere zusätzliche Peroxoverbindungen eingesetzt werden können.

Die Auswahl dieser weiteren Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Bevorzugte erfindungsgemäße Mittel zum Färben und/oder Aufhellen keratinischer Fasern sind dadurch gekennzeichnet, daß die fließfähige Oxidationsmittelzubereitung B zusätzlich mindestens eine Peroxoverbindung enthält, die vorzugsweise ausgewählt ist aus Ammonium- und Alkalimetallpersulfaten und -peroxidisulfaten, wobei bevorzugte Mittel mindestens 2 verschiedene Peroxidisulfate enthalten.

Die Peroxoverbindungen sind in den erfindungsgemäßen Blondiermitteln bevorzugt in Mengen von 5 bis 50 Gew.%, vorzugsweise von 10 bis 45 Gew.%, besonders bevorzugt von 15 bis 42,5 Gew.% und insbesondere von 20 bis 40 Gew.%, jeweils bezogen auf das gesamte Mittel, enthalten.

Als weitere Komponente können die erfindungsgemäßen Blondiermittel ein Alkalisierungsmittel enthalten, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxyde, -carbonate, - hydrogencarbonate, -hydroxycarbonate, -silikate, insbesondere -metasilikate, sowie Alkaliphosphate verwendet werden. In einer bevorzugten Ausführungsform enthalten die erfindungsgemäßen Blondierpulver mindestens zwei unterschiedliche Alkalisierungsmittel. Dabei können Mischungen beispielsweise aus einem Metasilikat und einem Hydroxycarbonat bevorzugt sein.

Die erfindungsgemäßen Blondiermittel enthalten Alkalisierungsmittel bevorzugt in Mengen von 5 - 30 Gew.-%, insbesondere von 15 - 25 Gew.-%.

Eine weitere bevorzugte Ausführungsform erfindungsgemäßer Mittel stellen die Färbemittel dar. Farbstoffe und Farbstoffvorprodukte, die in der Zubereitung A enthalten sein können, wurden weiter oben ausführlich beschrieben.

Zusätzlich können die erfindungsgemäßen Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäße Mittel bevorzugt, die zusätzlich Cu-, Fe-, Mn-, Ru-Ionen oder Komplexe dieser Ionen enthalten.

Bevorzugte erfindungsgemäße Haarfärbe- und - aufhellungsmittel enthalten 0,0001 bis 2,5 Gew.-%, vorzugsweise 0,001 bis 1 Gew.-%, bezogen auf die Gesamtzusammensetzung des Mittels, mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Kaliumjodid, Natriumjodid, Lithiumchlorid, Kaliumdichromat, Magnesiumacetat, Calciumchlorid, Calciumnitrat, Bariumnitrat, Mangandioxid (MnO₂) und/oder Hydrochinon.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Blondiermittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammonium-chlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, sowie auf die weiter unten stehenden Ausführungen verwiesen.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprcdukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Diese Tenside wurden weiter oben ausführlich beschrieben.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O-Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn Polymere (G) in den erfindungsgemäßen Mitteln enthalten sind. In einer bevorzugten Ausführungsform werden den erfindungsgemäßen Mitteln daher Polymere zugesetzt, wobei sich sowohl kationische, anionische, amphotere als auch nichtionische Polymere als wirksam erwiesen haben.

Erfindungsgemäß einsetzbar sind vorzugsweise kationische bzw. amphotere Polymere. Unter kationischen bzw. amphoteren Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 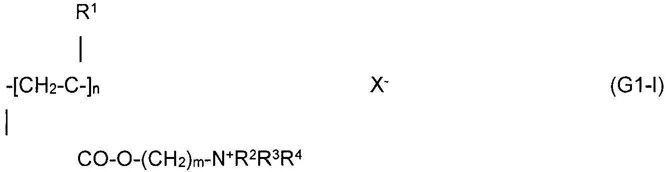 in der R¹= -H oder -CH₃ ist, R², R³ und R⁴ unabhängig voneinander ausgewählt sind aus C1-4-Alkyl-, -Alkenyl- oder-Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Zusätzlich zu kationischen Polymerisaten oder an ihrer Stelle können die erfindungsgemäßen Mittel auch amphotere Polymere enthalten. Diese weisen zusätzlich mindestens eine negativ geladene Gruppe im Molekül auf und werden auch als zwitterionische Polymere bezeichnet. Im Rahmen der vorliegenden Erfindung bevorzugt einsetzbare zwitterionische Polymerisate setzen sich im wesentlichen zusammen aus
A) Monomeren mit quartären Ammoniumgruppen der allgemeinen Formel (Z-I),

   R¹-CH=CR²-CO-Z-(CₙH₂ₙ)-N⁽⁺⁾R³R⁴R⁵ A⁽⁻⁾ (Z-I)

   In der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
B) monomeren Carbonsäuren der allgemeinen Formel (Z-II),

   R⁶-CH=CR⁷-COOH (Z-II)

   in denen R⁶ und R⁷ unabhängig voneinander Wasserstoff oder Methylgruppen sind.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quaternisierungsreaktion kann in wäßriger Lösung oder im Lösungsmittel erfolgen.

Vorteilhafterweise werden solche Monomere der Formel (Z-I), die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (Z-I), bei denen R³, R⁴ und R⁵ Methylgruppen sind.

Das Acrylamidopropyl-trimethylammoniumchlorid ist ein ganz besonders bevorzugtes Monomer der Formel (Z-I).

Als monomere Carbonsäuren der Formel (Z-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (Z-I) und (Z-II) nach an sich bekannten Polymerisationsverfahren hergestellt. Die Polymerisation kann entweder in wäßriger oder wäßrig-alkoholischer Lösung erfolgen. Als Alkohole werden Alkohole mit 1 bis 4 Kohlenstoffatomen, vorzugsweise Isopropanol, verwendet, die gleichzeitig als Polymerisationsregler dienen. Der Monomerlösung können aber auch andere Komponenten als Regler zugesetzt werden, z. B. Ameisensäure oder Mercaptane, wie Thioethanol und Thioglykolsäure. Die Initiierung der Polymerisation erfolgt mit Hilfe von radikalbildenden Substanzen. Hierzu können Redoxsysteme und/oder thermisch zerfallende Radikalbildner vom Typ der Azoverbindungen, wie z. B. Azoisobuttersäurenitril, Azo-bis-(cyanopentansäure) oder Azo-bis-(amidinopropan)dihydrochlorid verwendet werden. Als Redoxsysteme eignen sich z. B. Kombinationen aus Wasserstoffperoxid, Kalium- oder Ammoniumperoxodisulfat sowie tertiäres Butylhydroperoxid mit Natriumsulfit, Natriumdithionit oder Hydroxylaminhydrochlorid als Reduktionskomponente.

Die Polymerisation kann isotherm oder unter adiabatischen Bedingungen durchgeführt werden, wobei in Abhängigkeit von den Konzentrationsverhältnissen durch die freiwerdende Polymerisationswärme der Temperaturbereich für den Ablauf der Reaktion zwischen 20 und 200 °C schwanken kann, und die Reaktion gegebenenfalls unter dem sich einstellenden Überdruck durchgeführt werden muß. Bevorzugterweise liegt die Reaktionstemperatur zwischen 20 und 100 °C.

Der pH-Wert während der Copolymerisation kann in einem weiten Bereich schwanken. Vorteilhafterweise wird bei niedrigen pH-Werten polymerisiert; möglich sind jedoch auch pH-Werte oberhalb des Neutralpunktes. Nach der Polymerisation wird mit einer wäßrigen Base, z. B. Natronlauge, Kalilauge oder Ammoniak, auf einen pH-Wert zwischen 5 und 10, vorzugsweise 6 bis 8, eingestellt. Nähere Angaben zum Polymerisationsverfahren können den Beispielen entnommen werden.

Als besonders wirksam haben sich solche Polymerisate erwiesen, bei denen die Monomeren der Formel (Z-I) gegenüber den Monomeren der Formel (Z-II) im Überschuß vorlagen. Es ist daher erfindungsgemäß bevorzugt, solche Polymerisate zu verwenden, die aus Monomeren der Formel (Z-I) und die Monomeren der Formel (Z-II) in einem Molverhältnis von 60:40 bis 95:5, insbesondere von 75:25 bis 95:5, bestehen.

Die kationischen bzw. amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze^{®} QM im Handel erhältlich.

Die erfindungsgemäßen Mittel können in einer weiteren Ausführungsform nichtionogene Polymere (G4) enthalten.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone.

Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten.

Die Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methansulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20°C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird. Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylamino-benzoesäureamid ableiten, sind erfindungsgemäß bevorzugt.

Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)x-N⁺R¹R²R³ X⁻, in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X- für ein physiologisch verträgliches Anion. Im Rahmen dieser allgemeinen Struktur steht x bevorzugt für die die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyldimethylaminobenzamidopropyl-dimethylammoniumtosylat (Escalol^{®} HP 610).

Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den erfindungsgemäßen Mitteln üblicherweise in Mengen 0,1-5 Gew.-%, bezogen auf das gesamte Mittel, enthalten. Mengen von 0,4-2,5 Gew.-% sind bevorzugt.

Die erfindungsgemäßen Mittel können weiterhin eine 2-Pyrrolidinon-5-carbonsäure und deren Derivate (J) enthalten. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.%.

Schließlich können die erfindungsgemäßen Mittel auch Pflanzenextrakte (L) enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Vorteilhaft im Sinne der Erfindung können zusätzlich kurzkettige Carbonsäuren (N) den Wirkstoffkomplex (A) unterstützen. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäß verwendeten Carbonsäuren können selbstverständlich entlang der Kohlenstoffkette oder des Ringgerüstes substituiert sein. Zu den Substituenten der erfindungsgemäß verwendeten Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in □ - Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxyphthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 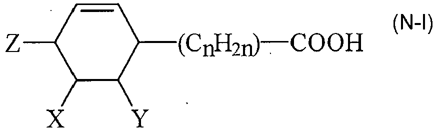 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten erfindungsgemäß verwendeten kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-,-ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren gemeinsam mit dem Wirkstoff (A) einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Eine besonders bevorzugte Gruppe von Inhaltsstoffen stellen die Silikone dar.

Erfindungsgemäße bevorzugte Mittel sind dadurch gekennzeichnet, daß sie mindestens ein Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
oder deren Gemischen.

Erfindungsgemäß besonders bevorzugte Färbemittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Bevorzugte Silikone werden nachstehend beschrieben.

Besonders bevorzugte erfindungsgemäße Oxidationsfärbemittel sind dadurch gekennzeichnet, daß sie mindestens ein Silikon der Formel Si-I

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Diese Silikone werden nach der INCI-Nomenklatur als DIMETHICONE bezeichnet. Es werden im Rahmen der vorliegenden Erfindung als Silicon der Formel Si-I vorzugsweise die Verbindungen:

(CH₃)₃Si-O-Si(CH₃)₃

(CH₃)₃Si-O-(CH₃)₂Si-O-Si(CH₃)₃

(CH₃)₃Si-[O-(CH₃)₂Si]₂-O-Si(CH₃)₃

(CH₃)₃Si-[O-(CH₃)₂Si]₃-O-Si(CH₃)₃

(CH₃)₃Si-[O-(CH₃)₂Si]₄-O-Si(CH₃)₃

(CH₃)₃Si-[O-(CH₃)₂Si]₅-O-Si(CH₃)₃

(CH₃)₃Si-[O-(CH₃)₂Si]₆-O-Si(CH₃)₃

(CH₃)₃Si-[O-(CH₃)₂Si]₇-O-Si(CH₃)₃

(CH₃)₃Si-[O-(CH₃)₂Si]₈-O-Si(CH₃)₃

(CH₃)₃Si-[O-(CH₃)₂Si]₉-O-Si(CH₃)₃

(CH₃)₃Si-[O-(CH₃)₂Si]₁₀-O-Si(CH₃)₃

(CH₃)₃Si-[O-(CH₃)₂Si]₁₁-O-Si(CH₃)₃

(CH₃)₃Si-[O-(CH₃)₂Si]₁₂-O-Si(CH₃)₃

(CH₃)₃Si-[O-(CH₃)₂Si]₁₃-O-Si(CH₃)₃

(CH₃)₃Si-[O-(CH₃)₂Si]₁₄-O-Si(CH₃)₃

(CH₃)₃Si-[O-(CH₃)₂Si]₁₅-O-Si(CH₃)₃

(CH₃)₃Si-[O-(CH₃)2Si]₁₆-O-Si(CH₃)₃

(CH₃)₃Si-[O-(CH₃)₂Si]₁₇-O-Si(CH₃)₃

(CH₃)₃Si-[O-(CH₃)₂Si]₁₈-O-Si(CH₃)₃

(CH₃)₃Si-[O-(CH₃)₂Si]₁₉-O-Si(CH₃)₃

(CH₃)₃Si-[O-(CH₃)₂Si]₂₀-O-Si(CH₃)₃

eingesetzt, wobei (CH₃)₃Si--O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silikone in den erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Besonders bevorzugte erfindungsgemäße Mittel enthalten ein oder mehrere aminofunktionelle Silicone. Solche Silicone können z.B. durch die Formel

M(RₐQ_{b}SiO_{(4-a-b)/2})ₓ(R_{c}SiO_{(4-c)/2})_{y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen,-CH₂CH(CH₃)CH₂-, Phenylen, Naphthylen, -CH₂CH₂SCH₂CH₂-, -CH₂CH₂OCH₂-, -OCH₂CH₂-,-OCH₂ CH₂CH₂-, -CH₂CH(CH₃)C(O)OCH₂-, -(CH₂)₃ CC(O)OCH₂CH₂-, -C₆H ₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Oxidationsfärbemittel sind dadurch gekennzeichnet, daß sie ein aminofunktionelles Silikon der Formel (Si-II)

R'ₐG₃₋ₐ-Si(OSiG₂)ₙ-(OSiG_{b}R'_{2-b})ₘ-O-SiG₃₋ₐ-R'ₐ (Si-II),

enthalten, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃,-CH₂CH₂CH₂CH₃, -O-CH₂CH(CH₃)₂, -CH₂CH(CH₃)₂, -O-CH(CH₃)CH₂CH₃,-CH(CH₃)CH₂CH₃, -O-C(CH₃)₃, -C(CH₃)₃;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂ A-
   ∘ -Q-N⁺H₂(R")A⁻
   ∘ -Q-N(R")-CH₂-CH₂-N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃,-CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, daß sie mindestens ein aminofunktionelles Silikon der Formel (Si-IIa) 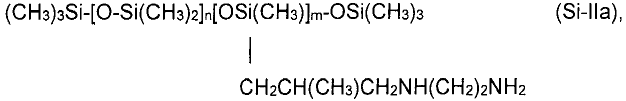 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch erfindungsgemäße Mittel, die ein aminofunktionelles Silikon der Formel (Si-IIb) 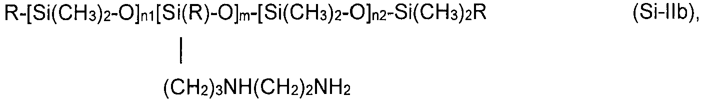 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Erfindungsgemäß bevorzugte Färbemittel sind dadurch gekennzeichnet, daß sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind erfindungsgemäße Färbemittel für keratinische Fasern bevorzugt, die mindestens ein Silikon der Formel Si-III 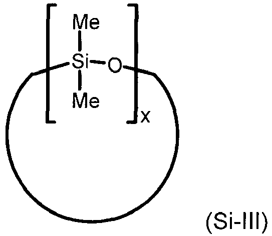 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Die erfindungsgemäß einsetzbaren Silikon-in-Wasser Emulsionen können nach bekannten Verfahren hergestellt werden, wie sie beispielsweise in US 5,998,537 und EP 0 874 017 A1 offenbart sind.

Zusammenfassend umfaßt dieses Herstellungsverfahren die emulgierende Mischung von Komponenten, deren eine mindestens ein Polysiloxane enthält, deren andere mindestens ein Organosilikonmaterial enthält, das mit dem Polysiloxane in einer Kettenverlängerungsreaktion reagiert, wobei mindestens ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]n-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Sigebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, daß durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, welche Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), vorzugsweise sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Si-haltige Gruppen, O-haltige Gruppen usw.. Vorzugsweise sind mindestens 80% der Reste R Alkylreste, besonders bevorzugt Methylgruppen.

Das Organosilikonmaterial, das mit dem Polysiloxan in der Kettenverlängerungsreaktion reagiert, kann entweder ein zweites Polysiloxan sein, oder ein Molekül, das als Kettenverlängerer agiert. Wenn das Organosilikonmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.

Falls das Organosilikonmaterial ein Kettenverlängerungs-Agens umfaßt, kann dies ein Material sein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen generellen Struktur umfaßt, welches mindestens eine Si-OH Gruppe aufweist, kettenverlängert werden, indem mit einem Alkoxysilan (beispielsweise einem Dialkoxysilan oder Trialkoxysilan) in Gegenwart von Zinn- oder Titan-haltigen Katalysatoren reagiert wird.

Die metallhaltigen Katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem Organosilikonmaterial in Gegenwart eines Hydrosyslylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vorstehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das Organosilikonmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n 0 oder eine positive ganze Zahl ist

Dieses Material kann ein Polymer oder ein niedermolekulares Material wie ein Siloxan sein (beispielsweise ein Disiloxane oder ein Trisiloxan).

Das Polysiloxan mit mindestens einer aliphatisch ungesättigten Gruppe und das Organosilikonmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin- oder Chloroplatinsäuren. Ein wegen der guten Dispergierbarkeit in Organosilikonsystemen und der geringen Farbveränderungen bevorzugter Katalysator ist Chloroplatinsäure entweder als kommerziell verfügbares Hexahydrat oder in wasserfreier Form.

Bei einer weiteren bevorzugten Kettenerweiterungsreaktion wird ein Polysiloxan mit mindestens einer Si-OH Gruppe, vorzugsweise einer Endgruppe, mit einem Organosilikonmaterial zu Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.

Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinn-octoat, Dibutylzinn-dilaurat, Dibutylzinndiacetat, Dimethylzinn-dineodecanoat, Dibutylzinn-dimethoxid, Isobutylzinn-triceroat, Dimethylzinn-dibutyrat, Dimethylzinn-dineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitante, Tetraphenyltitanat, Tetraoctadecyltitanat, Titan-naphthanat, Ethyltriethanolamin-Titanat, Titani-diisopropyl-diethyl-acetoacetat, Titan-diisopropoxy-diacetylacetonat und Titani-tetra-Alkoxide, bei denen das Alkoxid Butoxy oder Propoxy ist.

Erfindungsgemäß ebenfalls bevorzugte Färbemittel sind dadurch gekennzeichnet, daß sie mindestens ein Silikon der Formel Si-IV

R₃Si-[O-SiR₂]ₓ-(CH₂)ₙ-[O-SiR₂]_{y}-O-SiR₃ (Si-IV),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃,-CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Färbemittel sind dadurch gekennzeichnet, daß sie mindestens ein wasserlösliches Silikon enthalten.

Bei einer Anwendung von Oxidationsmitteln wird das eigentliche Färbemittel wie weiter oben erwähnt, zweckmäßigerweise unmittelbar vor der Anwendung durch Mischung der Zubereitung des Oxidationsmittels mit der Zubereitung, enthaltend die Verbindungen a) und b) und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein weiterer Gegenstand der vorliegenden Erfindung ist ein Verfahren zum Färben und/oder Aufhellen keratinischer Fasern, bei dem
- gewünschtenfalls ein Vorbehandlungsmittel M1 auf die Faser aufgebracht wird, dann
- ein Tönungs- oder Färbemittel M2 auf der Faser zur Anwendung kommt, wobei gewünschtenfalls dem Mittel M2 vor der Anwendung ein weiteres Mittel M3 zugegeben wird,
- dieses Färbemittel M2 nach einer Zeit von 5-45 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
dadurch gekennzeichnet, daß mindestens eines der Mittel M1, M2 oder M3 ein erfindungsgemäßes Mittel ist.

In erfindungsgemäß bevorzugten Verfahren wird das Haar im Anschluß an die Appilkation des ersten Mittels ausgespült, und besonders bevorzugte Verfahren sind dadurch gekennzeichnet, daß die Applikation von Haarfärbemittel(n) auf feuchtes Haar oder die feuchten Haarkonturen erfolgt. "Feucht" im Sinne der vorliegenden Anmeldung ist auch handtuchtrocken.

Bezüglich bevorzugter Ausführungsformen des erfindungsgemäßen Verfahrens gilt mutatis mutandis das zu den erfindungsgemäßen Mitteln Ausgeführte. Insbesondere sind erfindungsgemäße Verfahren bevorzugt, bei denen das Haarfärbemittel mindestens einen direktziehenden Farbstoff enthält.

Auch erfindungsgemäße Verfahren, bei denen das Haarfärbemittel mindestens eine aromatische oder heteroaromatische aktivierte Carbonylverbindung und/oder eine CH-acide Verbindung enthält, sind bevorzugte Ausführungsformen der vorliegenden Erfindung.

### Beispiele:

Es wurde eine Blondiercreme folgender Zusammensetzung (Angaben in Gew.-%) hergestellt:

| | |
|---|---|
| C₁₆₋₁₈-Fettalkohol | 10,0 |
| C₁₂₋₁₈-Kokosfettalkohol | 3,0 |
| Eumulgin^{®} B2* | 3,0 |
| Ammoniumsulfat | 1,0 |
| Turpinal^{®} SL** | 0,2 |
| Gluadin W 40*** | 6,5 |
| Parfümöl | 0,4 |
| Valin | 1,0 |
| Wasser | ad 100 |
| Mit Ammoniaklösung 25% auf pH = 10,0 | |

| | |
|---|---|
| * Cetylstearylalkohol mit ca. 20 EO-Einheiten (INCI-Bezeichnung: Ceteareth-20) (Cognis) ** 1-Hydroxyethan-1,1-diphosphonsäure (60 % Aktivsubstanz; INCI - Bezeichnung: Etidronic Acid) (COGNIS) *** Partialhydrolysat aus Weizen ca. 40% Aktivsubstanz (INCI - Bezeichnung: Hydrolyzed Wheat Gluten Hydrolyzed Wheat Protein) (COGNIS) **** N-Succinyl-Chitosan ca. 1,5 Gew.-% Aktivsubstanz (INCI - Bezeichnung: Chitosan Succinamide) (Impag) | |

Zusätzlich wurde eine Entwickleremulsion folgender Zusammensetzung (Angaben in Gew.-%) hergestellt:

| | |
|---|---|
| C₁₆-Fettalkohol | 3,6 |
| Eumulgin^{®} B2 | 0,9 |
| Disponil^{®} FES 77 IS* | 2,25 |
| Wasserstoffperoxid, 30% | 11,2 |
| Turpinal^{®} SL | 1,5 |
| Aculyn^{®} 33 A** | 15,0 |
| Wasser | ad 100 |
| Mit Ammoniaklösung 25% auf pH = 3,8 - 4,2 | |

| | |
|---|---|
| * Natriumlaurylethersulfat (Cognis) ** Säure-enthaltendes, vernetztes Acrylcopolymer (INCI-Bezeichnung: Acrylates Copolymer, ca. 28% Aktivsubstanz) (Rohm&Haas) | |

Blondiercreme und Entwickleremulsion wurden im Verhältnis 1:1 vermischt und an der Chorionallantoismembran (CAM) befruchteter und neun Tage bebrüteter Hühnereier auf ihre Reizpotentiale untersucht. Die Untersuchung erfolgte durch Endpunktbeurteilung mit einer Expositionszeit von 30 Sekunden, wobei die Exposition 30 Sekunden nach Applikation der Mischung durch Abspülen mit physiologischer Kochsalzlösung beendet wurde. Nach weiteren 30 Sekunden wurde das Substrat auf
- Hämorraghie (Blutung aus Gefäßen)
- Koagulation (Proteingerinnung extravasal und/oder intravasal)
- Lysierung (Auflösung der Gefäßwände)
untersucht.

Zur Korrektur biologischer Schwankungen in der Eichcharge wurde parallel der Reizindex bzw. das relative Reizpotential des Prüfsubstanzen im Vergleich zur Referenzsubstanz "Texapon ASV" (5%ige Lösung von Laurylethersulfaten in Wasser, INCI: Sodium Laureth Sulfate (and) Sodium. Laureth 8-Sulfate (and) Magnesium Laureth. Sulfate (and) Magnesium Laureth 8-Sulfate) ermittelt.

Die Bewertung erfolgte anhand folgender Skala:

| Reaktionszeitmethode | Endpunktbeurteilung | Beurteilung |
|---|---|---|
| ≤ 0,8 | 0 bis 5 | gering reizend |
| > 0,8 bis < 1,2 | 6 bis 12 | mäßig reizend |
| ≥ 1,2 bis < 2,0 | 13 bis 15 | reizend |
| ≥ 2,0 | 16 bis 18 | stark reizend |

Das erfindungsgemäße Produkt wurde mit "gering reizend" beurteilt, während Produkte, in denen entweder das Valin (V1) oder das Proteinhydrolysat (V2) durch Wasser ersetzt war, als "mäßig reizend" beurteilt wurden.

## Patentansprüche

1. Mittel zum Färben und/oder Aufhellen keratinischer Fasern, insbesondere menschlicher Haare, das, bezogen auf sein Gewicht
a) 0,05 bis 10 Gew.-% Valin;
b) 0,01 bis 10 Gew.-% Proteinhydrolysat(e)
enthält,
**dadurch gekennzeichnet, dass** das/die Proteinhydrolysat(e) Molmassen im Bereich von 250 bis 50.000 Dalton aufweisen.

2. Mittel zum Färben und/oder Aufhellen keratinischer Fasern nach Anspruch 1, **dadurch gekennzeichnet, daß** es - bezogen auf sein Gewicht - 0,1 bis 7,5 Gew.-%, vorzugsweise 0,15 bis 5 Gew.-%, besonders bevorzugt 0,25 bis 3,5 Gew.-% und insbesondere 0,5 bis 2 Gew.-% Valin enthält.

3. Mittel zum Färben und/oder Aufhellen keratinischer Fasern nach einem der Ansprüche 1 oder 2, **dadurch gekennzeichnet, daß** es - bezogen auf sein Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 1,5 Gew.-% Proteinhydrolysat(e) enthält.

4. Mittel zum Färben und/oder Aufhellen keratinischer Fasern nach einem der Ansprüche 1 bis 3, **dadurch gekennzeichnet, daß** das/die Proteinhydrolysat(e) Molmassen im Bereich von 500 bis 20.000 Dalton aufweisen.

5. Mittel zum Färben und/oder Aufhellen keratinischer Fasern nach einem der Ansprüche 1 bis 4, **dadurch gekennzeichnet, daß** das/die in ihm enthaltene(n) Proteinhydrolysat(e) ausgewählt ist/sind aus Hydrolysaten von tierischen und/oder pflanzlichen Proteinen, vorzugsweise aus
- Milchproteinhydrolysaten
- Keratinhydrolysaten
- Weizenproteinhydrolysaten
- Maisproteinhydrolysaten
- Reisproteinhydrolysaten
- Kartoffelproteinhydrolysaten
- Sojaproteinhydrolysaten
- Mandelproteinhydrolysaten
- Algenproteinhydrolysaten.

6. Mittel zum Färben und/oder Aufhellen keratinischer Fasern nach einem der Ansprüche 1 bis 5, **dadurch gekennzeichnet, daß** es kationische Proteinhydrolysate enthält, wobei bevorzugte Mittel ein oder mehrere kationische Proteinhydrolysate aus der Gruppe Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein enthalten.

7. Mittel zum Färben und/oder Aufhellen keratinischer Fasern nach einem der Ansprüche 1 bis 6, **dadurch gekennzeichnet, daß** es zusätzlich Vitamine und/oder Pro-Vitamine und/oder Vitaminvorstufen enthält, die vorzugsweise den Gruppen A, B, C, E, F und H zugeordnet werden, wobei bevorzugte Mittel die genannten Verbindungen in Mengen von von 0,1 bis 5 Gew.-%, vorzugsweise von 0,25 bis 4 Gew.-% und insbesondere von 0,5 bis 2,5 Gew.-%, jeweils bezogen auf das gesamte Mittel, enthalten.

8. Mittel zum Färben und/oder Aufhellen keratinischer Fasern nach einem der Ansprüche 1 bis 7, **dadurch gekennzeichnet, daß** es zusätzlich 0,05 bis 15 Gew.-% mindestens eines 2-Furanonderivats der Formel (I) und/oder der Formel (II) 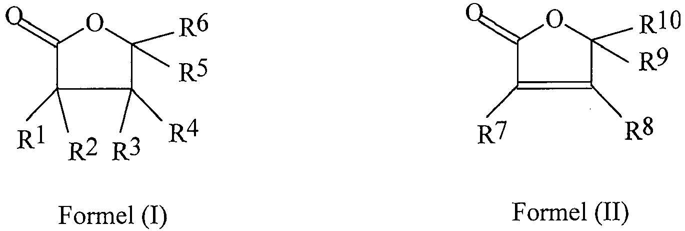 enthält, in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂-C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen-C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄-gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀-gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

9. Mittel zum Färben und/oder Aufhellen keratinischer Fasern nach einem der Ansprüche 1 bis 8, **dadurch gekennzeichnet, daß** es zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol 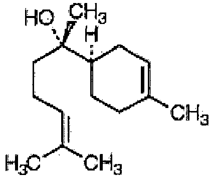 enthält.

10. Mittel zum Färben und/oder Aufhellen keratinischer Fasern, insbesondere menschlicher Haare, welches unmittelbar vor dem Aufbringen auf das Haar aus einer fließfähigen Zubereitung A und einer Oxidationsmittelzubereitung B, enthaltend mindestens ein Oxidationsmittel, ausgewählt aus Wasserstoffperoxid und dessen Anlagerungsverbindungen an feste Träger, die miteinander im Gewichtsverhältnis 1:4 bis 4:1 zu einem Färbe- und/oder Aufhellansatz vermischt werden, erhalten wird, **dadurch gekennzeichnet, daß** die fließfähige Zubereitung A - bezogen auf ihr Gewicht -
a. 0,1 bis 20 Gew.-% Valin;
b. 0,02 bis 20 Gew.-% Proteinhydrolysat(e)
enthält.

11. Verfahren zum Färben und/oder Aufhellen keratinischer Fasern, bei dem
- gewünschtenfalls ein Vorbehandlungsmittel M1 auf die Faser aufgebracht wird, dann
- ein Tönungs- oder Färbemittel M2 auf der Faser zur Anwendung kommt, wobei gewünschtenfalls dem Mittel M2 vor der Anwendung ein weiteres Mittel M3 zugegeben wird,
- dieses Färbemittel M2 nach einer Zeit von 5-45 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird, **dadurch gekennzeichnet, daß** mindestens eines der Mittel M1, M2 oder M3 ein Mittel nach einem der Ansprüche 1 bis 9 ist.

## Claims

1. An agent for coloring and/or lightening keratin fibers, in particular human hair, which agent contains, based on its weight
a) 0.05 to 10 wt.% valine;
b) 0.01 to 10 wt.% protein hydrolysate(s),
**characterized in that** the protein hydrolysate(s) have molar masses in the range of from 250 to 50,000 Dalton.

2. The agent for coloring and/or lightening keratin fibers according to claim 1, **characterized in that** it contains, based on its weight, 0.1 to 7.5 wt.%, preferably 0.15 to 5 wt.%, particularly preferably 0.25 to 3.5 wt.% and in particular 0.5 to 2 wt.% valine.

3. The agent for coloring and/or lightening keratin fibers according to either claim 1 or 2, **characterized in that** it contains, based on its weight, 0.05 to 7.5 wt.%, preferably 0.1 to 5 wt.%, particularly preferably 0.2 to 3.5 wt.% and in particular 0.25 to 1.5 wt.% protein hydrolysate(s).

4. The agent for coloring and/or lightening keratin fibers according to one of claims 1 to 3, **characterized in that** the protein hydrolysate(s) have molar masses in the range of from 500 to 20,000 Dalton.

5. The agent for coloring and/or lightening keratin fibers according to one of claims 1 to 4, **characterized in that** the protein hydrolysate(s) contained in said agent is/are selected from hydrolysates of animal and/or plant proteins, preferably from
- milk protein hydrolysates
- keratin hydrolysates
- wheat protein hydrolysates
- maize protein hydrolysates
- rice protein hydrolysates
- potato protein hydrolysates
- soya protein hydrolysates
- almond protein hydrolysates
- algae protein hydrolysates.

6. The agent for coloring and/or lightening keratin fibers according to one of claims 1 to 5, **characterized in that** it contains cationic protein hydrolysates, preferred agents containing one or more cationic protein hydrolysates from the group of Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, HydroxypropyltrimoniumHydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

7. The agent for coloring and/or lightening keratin fibers according to one of claims 1 to 6, **characterized in that** it additionally contains vitamins and/or provitamins and/or vitamin precursors, which are preferably assigned to the groups A, B, C, E, F and H, preferred agents containing the aforementioned compounds in amounts of from 0.1 to 5 wt.%, preferably from 0.25 to 4 wt.% and in particular from 0.5 to 2.5 wt.%, in each case based on the total agent.

8. The agent for coloring and/or lightening keratin fibers according to one of claims 1 to 7, **characterized in that** it additionally contains 0.05 to 15 wt.% of at least one 2-furanone derivative of Formula (I) and/or Formula (II), 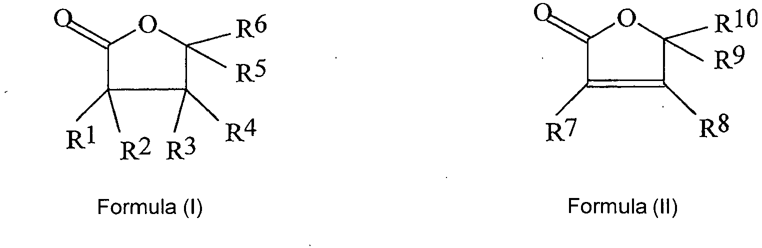 where the functional groups R¹ to R¹⁰ represent, independently of one another:
- hydrogen, -OH, a methyl, methoxy, aminomethyl or hydroxymethyl functional group,
- -C₂ - C₄ - saturated or mono- or diunsaturated, branched or linear hydrocarbon functional group,
- -C₂ - C₄ - saturated or mono- or diunsaturated, branched or linear mono-, di- or trihydroxy hydrocarbon functional group,
- -C₂ - C₄ - saturated or mono-or diunsaturated, branched or linear mono-, di- or triamino hydrocarbon functional group,
- an -OR¹¹ group, where R¹¹ is a -C₂ - C₄ - saturated or mono- or diunsaturated branched or linear hydrocarbon functional group, -C₂ - C₄ - saturated or mono- or diunsaturated, branched or linear mono-, di- or trihydroxy hydrocarbon functional group,
- an -NR¹²R¹³ group, where R¹² and R¹³ each represent, independently of one another, hydrogen, a methyl-, a -C₂ - C₄ - saturated or mono- or diunsaturated, branched or linear hydrocarbon functional group, a -C₂ - C₄ - saturated or mono- or diunsaturated, branched or linear mono-, di- or trihydroxy hydrocarbon functional group,
- a -COOR¹⁴ group, where R¹⁴ represents hydrogen, a methyl-, a -C₂ - C₄ saturated or mono- or diunsaturated, branched or linear hydrocarbon functional group, a -C₂ - C₄-saturated mono- or diunsaturated, branched or linear mono-, di- or trihydroxy hydrocarbon functional group, a -C₂ - C₄ - saturated or mono- or diunsaturated, branched or linear mono-, di- or triamino hydrocarbon functional group,
- a -CONR¹⁵R¹⁶ group, where R¹⁵ and R¹⁶ each represent hydrogen, a methyl-, a -C₂ -C₄ saturated or mono- or diunsaturated, branched or linear hydrocarbon functional group, a -C₂ - C₄ - saturated mono- or diunsaturated, branched or linear mono-, di- or trihydroxy hydrocarbon functional group, a -C₂ - C₄ - saturated or mono- or diunsaturated, branched or linear mono-, di- or triamino hydrocarbon functional group,
- a -COR¹⁶ group, where R¹⁶ represents a methyl-, a -C₂ - C₄ - saturated or mono- or diunsaturated, branched or linear hydrocarbon functional group, a -C₂ - C₄ - saturated or mono- or diunsaturated, branched or linear mono-, di- or trihydroxy hydrocarbon functional group, a -C₂ - C₄ - saturated or mono- or diunsaturated, branched or linear mono-, di- or triamino hydrocarbon functional group,
- an -OCOR¹⁷ group, where R¹⁷ represents a methyl-, a -C₂ - C₃₀ - saturated or mono- or polyunsaturated, branched or linear hydrocarbon functional group, a -C₂ -C₃₀-saturated or mono- or polyunsaturated, branched or linear mono-, di-, tri- or polyhydroxy hydrocarbon functional group, a -C₂ - C₃₀ - saturated or mono- or polyunsaturated, branched or linear mono-, di-, tri- or polyamino hydrocarbon functional group,
with the proviso that when R⁷ and R⁸ represent -OH and at the same time R⁹ or R¹⁰ represents hydrogen, the remaining R⁹ or R¹⁰ group does not represent a dihydroxyethyl functional group.

9. The agent for coloring and/or lightening keratin fibers according to one of claims 1 to 8, **characterized in that** it additionally contains 0.001 to 5 wt.%, preferably 0.01 to 4 wt.%, particularly preferably 0.02 to 2.5 wt.% and in particular 0.1 to 1.5 wt.% bisabolol and/or oxides of bisabolol, preferably (-)-alpha-bisabolol.

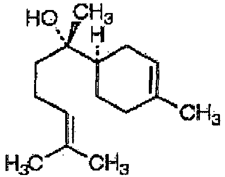

10. The agent for coloring and/or lightening keratin fibers, in particular human hair, which, immediately before being applied to the hair, is obtained from a flowable preparation A and an oxidizing agent preparation B, containing at least one oxidizing agent, selected from hydrogen peroxide and the addition compounds thereof on solid carriers, which are intermixed in a 1:4 to 4:1 weight ratio to form a coloring and/or lightening formulation, **characterized in that** the flowable preparation A contains, based on its weight,
a) 0.1 to 20 wt.% valine;
b) 0.02 to 20 wt.% protein hydrolysate(s).

11. A method for coloring and/or lightening keratin fibers, in which
- a pretreatment agent M1 is applied to the fiber if desired, then
- a tinting or coloring agent M2 is used on the fiber, an additional agent M3 being added, if desired, to the agent M2 before said agent is used,
- said coloring agent M2 is rinsed off the fiber after 5-45 minutes,
- and a post-treatment agent M4 is optionally applied to the fiber after treatment and is rinsed off again after a contact time of several minutes,
**characterized in that** at least one of the agents M1, M2 or M3 is an agent according to one of claims 1 to 9.

## Revendications

1. Produit de coloration et/ou d'éclaircissement de fibres de kératine, en particulier de cheveux humains, contenant, rapporté à son poids :
a) 0,05 à 10 % en poids de valine ;
b) 0,01 à 10 % en poids d'hydrolysat(s) protéinique(s),
**caractérisé en ce que** le(s) hydrolysat(s) protéinique(s) présentent des poids molaires entre 250 et 50 000 daltons.

2. Produit de coloration et/ou d'éclaircissement de fibres de kératine selon la revendication 1, **caractérisé en ce qu'**il contient, rapporté à son poids, 0,1 à 7,5 % en poids, de préférence 0,15 à 5 % en poids, particulièrement préférentiellement 0,25 à 3,5 % en poids, et en particulier 0,5 à 2 % en poids de valine.

3. Produit de coloration et/ou d'éclaircissement de fibres de kératine selon la revendication 1 ou 2, caractérisé en qu'il contient, rapporté à son poids, 0,05 à 7,5 % en poids, de préférence 0,1 à 5 % en poids, particulièrement préférentiellement 0,2 à 3,5 % en poids, et en particulier 0,25 à 1,5 % en poids d'hydrolysat(s) protéinique(s).

4. Produit de coloration et/ou d'éclaircissement de fibres de kératine selon la revendication 1 à 3, **caractérisé en ce que** le(s) hydrolysat(s) protéinique(s) présentent des poids molaires entre 500 et 20 000 daltons.

5. Produit de coloration et/ou d'éclaircissement de fibres de kératine selon la revendication 1 à 4, **caractérisé en ce que** le(s) hydrolysat(s) protéinique(s) qu'il contient est (sont) choisi(s) parmi des hydrolysats de protéines animales et/ou végétales, de préférence parmi :
- des hydrolysats de protéines de lait
- des hydrolysats de kératine
- des hydrolysats de protéines de blé
- des hydrolysats de protéines de maïs
- des hydrolysats de protéines de riz
- des hydrolysats de protéines de pomme de terre
- des hydrolysats de protéines de soja
- des hydrolysats de protéines d'amande
- des hydrolysats de protéines d'algues.

6. Produit de coloration et/ou d'éclaircissement de fibres de kératine selon la revendication 1 ou 5, caractérisé en qu'il contient des hydrolysats de protéines cationiques, des produits préférentiels contenant un ou plusieurs hydrolysats de protéines cationiques issus du groupe constitué de collagène hydrolysé de cocodimonium hydroxypropyle, caséine hydrolysé de cocodimonium hydroxypropyle, kératine capillaire hydrolysée de cocodimonium hydroxypropyle, kératine hydrolysée de cocodimonium hydroxypropyle, protéine de riz hydrolysée de cocodimonium hydroxypropyle, protéine de soja hydrolysée de cocodimonium hydroxypropyle, protéine de blé hydrolysée de cocodimonium hydroxypropyle, chlorhydrate d'hydroxypropyl-arginine-lauryl/mystyl-éther, gélatine trimonium hydroxypropyle, caséine hydrolysée trimonium hydroxypropyle, collagène hydrolysé trimonium hydroxypropyle, protéine de conchyoline hydrolysée trimonium hydroxypropyle, kératine hydrolysée trimonium hydroxypropyle, protéine de son de riz hydrolysée trimonium hydroxypropyle, protéine de soja hydrolysée trimonium hydroxypropyle, protéine végétale hydrolysée hydroxypropyle, protéine de blé hydrolysée trimonium hydroxypropyle, protéine de blé hydrolysée trimonium hydroxypropyle / siloxysilicate, protéine de soja hydrolysée laurmonium hydroxypropyle, protéine de blé hydrolysée laurmonium hydroxypropyle, protéine de blé hydrolysée laurdimonium hydroxypropyle, laurdimonium hydroxypropyle protéine de blé hydrolysée / siloxysilicate, caséine hydrolysée laurdimonium hydroxypropyle, collagène hydrolysé laurdimonium hydroxypropyle, kératine hydrolysée laurdimonium hydroxypropyle, protéine de soja hydrolysée laurdimonium hydroxypropyle, caséine hydrolysée stéardimonium hydroxypropyle, collagène hydrolysé stéardimonium hydroxypropyle, kératine hydrolysée stéardimonium hydroxypropyle, protéine de riz hydrolysée stéardimonium hydroxypropyle, protéine de soja hydrolysée stéardimonium hydroxypropyle, protéine végétale hydrolysée stéardimonium hydroxypropyle, protéine de blé hydrolysée stéardimonium hydroxypropyle, collagène hydrolysé stéartrimonium hydroxyéthyle, collagène hydrolysé quaternium-76, collagène hydrolysé quaternium-79, kératine hydrolysée quaternium-79, protéine de lait hydrolysée quaternium-79, protéine de soja hydrolysée quaternium-79, protéine de blé hydrolysée quaternium-79.

7. Produit de coloration et/ou d'éclaircissement de fibres de kératine selon la revendication 1 ou 6, caractérisé en qu'il contient en plus des vitamines et/ou des provitamines et/ou des précurseurs de vitamines, classés préférentiellement parmi les groupes A, B, C, E, F et H, les produits préférentiels contenant les composés spécifiés à hauteur de 0,1 à 5 % en poids, de préférence 0,25 à 4 % en poids et particulièrement préférentiellement 0,5 à 2,5 % en poids rapporté au produit total.

8. Produit de coloration et/ou d'éclaircissement de fibres de kératine selon la revendication 1 ou 7, caractérisé en qu'il contient en plus 0,05 à 15 % en poids d'au moins un dérivé de 2-furanone de formule (I) et/ou de formule (II) : 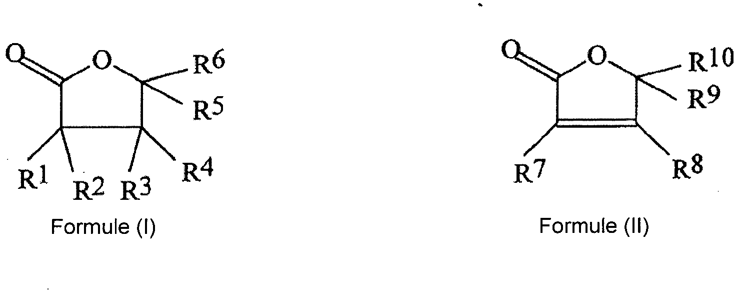 où les résidus R¹ à R¹⁰ représentent, indépendamment les uns des autres :
- hydrogène, OH, un résidu méthyle, méthoxy, aminométhyle ou hydroxyméthyle,
- un résidu C₂₋₄-hydrocarbure saturé ou une ou deux fois insaturé, linéaire ou ramifié,
- un résidu C₂₋₄-mono-, di- ou trihydroxyhydrocarbure saturé ou une ou deux fois insaturé, linéaire ou ramifié,
- un résidu C₂₋₄-mono-, di- ou triaminohydrocarbure saturé ou une ou deux fois insaturé, linéaire ou ramifié,
- un groupe -OR¹¹, où R¹¹ est un résidu C₂₋₄-hydrocarbure saturé ou une ou deux fois insaturé, linéaire ou ramifié, ou un résidu C₂₋₄-mono-, di- ou trihydroxyhydrocarbure saturé ou une ou deux fois insaturé, linéaire ou ramifié,
- un groupe -NR¹²R¹³, où R¹² et R¹³ représentent chacun, indépendamment l'un de l'autre, un résidu méthyle, un résidu C₂₋₄-hydrocarbure saturé ou une ou deux fois insaturé, linéaire ou ramifié, ou un résidu C₂₋₄-mono-, di- ou trihydroxyhydrocarbure saturé ou une ou deux fois insaturé, linéaire ou ramifié,
- un groupe -COOR¹⁴, où R¹⁴ représente un hydrogène, un résidu méthyle, un résidu C₂-₄-hydrocarbure saturé ou une ou deux fois insaturé, linéaire ou ramifié, un résidu C₂₋₄-mono-, di- ou trihydroxyhydrocarbure saturé ou une ou deux fois insaturé, linéaire ou ramifié, ou un résidu C₂₋₄-mono-, di- ou triaminohydrocarbure saturé ou une ou deux fois insaturé, linéaire ou ramifié,
- un groupe -CONR¹⁵R¹⁶, où R¹⁵ et R¹⁶ représentent chacun, indépendamment l'un de l'autre, un hydrogène, un résidu méthyle, un résidu C₂₋₄-hydrocarbure saturé ou une ou deux fois insaturé, linéaire ou ramifié, un résidu C₂₋₄-mono-, di- ou trihydroxyhydrocarbure saturé ou une ou deux fois insaturé, linéaire ou ramifié, ou un résidu C₂₋₄-mono-, di- ou triaminohydrocarbure saturé ou une ou deux fois insaturé, linéaire ou ramifié,
- un groupe -COR¹⁶, où R¹⁶ représente un résidu méthyle, un résidu C₂₋₄-hydrocarbure saturé ou une ou deux fois insaturé, linéaire ou ramifié, un résidu C₂₋₄-mono-, di- ou trihydroxyhydrocarbure saturé ou une ou deux fois insaturé, linéaire ou ramifié, ou un résidu C₂₋₄-mono-, di- ou triaminohydrocarbure saturé ou une ou deux fois insaturé, linéaire ou ramifié,
- un groupe -OCOR¹⁷, où R¹⁷ représente un résidu méthyle, un résidu C₂₋₃₀-hydrocarbure saturé ou une ou deux fois insaturé, linéaire ou ramifié, un résidu C₂₋₃₀-mono-, di-, tri- ou poly hydroxyhydrocarbure saturé ou une ou deux fois insaturé, linéaire ou ramifié, ou un résidu C₂₋₃₀-mono-, di-, tri- ou polyaminohydrocarbure saturé ou une ou deux fois insaturé, linéaire ou ramifié,
à condition que pour le cas où R⁷ et R⁸ représentent -OH et en même temps R⁹ ou R¹⁰ représentent un hydrogène, les groupes restant R⁹ ou R¹⁰ ne représentent pas un résidu dihydroxyéthyle.

9. Produit de coloration et/ou d'éclaircissement de fibres de kératine selon une des revendications 1 à 8, **caractérisé en ce qu'**il contient en plus 0,001 à 5 % en poids, de préférence 0,01 à 4 % en poids, particulièrement préférentiellement 0,02 à 2,5 % en poids et en particulier 0,1 à 1,5 % en poids de bisabolol et/ou d'oxyde de bisabolol, de préférence de (-)-α-bisabolol.

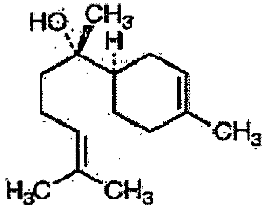

10. Produit de coloration et/ou d'éclaircissement de fibres de kératine, en particulier de cheveux humains, qui s'obtient immédiatement avant application sur les cheveux à partir d'une préparation A fluide et d'une préparation d'oxydant B contenant au moins un oxydant choisi parmi le peroxyde d'hydrogène et ses composés d'addition sur des supports solides, qu'on mélange l'une avec l'autre dans un rapport pondéral de 1:4 à 4:1 en une base de coloration et/ou d'éclaircissement, **caractérisé en ce que** la préparation fluide A contient, rapporté à son poids :
a) 0,1 à 20 % en poids de valine ;
b) 0,02 à 20 % en poids d'hydrolysat(s) de protéine.

11. Procédé de coloration et/ou d'éclaircissement de fibres de kératine, dans lequel :
- si on le désire, on applique un produit de préparation M1 sur les fibres, puis
- on utilise un produit de teinture ou de coloration M2 sur les fibres, où si on le désire, on ajoute au produit M2 un autre produit M3 avant utilisation,
- on rince ce produit colorant M2 après une durée de 5 à 45 minutes,
- et après traitement, éventuellement, on applique un produit complémentaire M4 sur les fibres, et on rince à nouveau après une durée de quelques minutes,
**caractérisé en ce qu'**au moins un des produits M1, M2 ou M3 est un produit selon une des revendications 1 à 9.
